# NEW EUROPEAN PATENT SPECIFICATION

(11) **EP 1 222 301 B2**
(45) Date of publication and mention of the opposition decision: **07.05.2008**
(45) Mention of the grant of the patent: 20.07.2005
(21) Application number: 00971523.6
(22) Date of filing: 19.10.2000
(51) Int. Cl.: C12N 15/90, C12N 15/54, C12N 9/22, A01K 67/033, C12P 21/02, C07K 14/61, C07K 14/47, C12N 9/24

(54) **PROTEIN PRODUCTION SYSTEM**
SYSTEM ZUR HERSTELLUNG VON PROTEINEN
SYSTEME DE PRODUCTION DE PROTEINE

(30) Priority: 19.10.1999 GB 9924721; 15.11.1999 US 165508 P
(43) Date of publication of application: 17.07.2002
(73) Proprietor: Minos Biosystems Limited, Smallwood, Sandbach, Cheshire CW11 2XB (GB)
(72) Inventor: CRAIG, Roger, Sandbach, Cheshire CW11 OXA (GB); SAVAKIS, Charalambos, 711 10 Heraklion (GR)
(74) Representative: Maschio, Antonio
(86) International application number: PCT/GB2000/004013
(87) International publication number: WO 2001/029204

(56) References cited:
- WO-A-90/01556
- WO-A-93/06224
- WO-A-98/44141
- US-A- 5 614 398
- US-A- 5 840 865
- DATABASE WPI Week 199718 Derwent Publications Ltd., London, GB; AN 1997-193471 XP002164516 QIN J ET AL: "Recombination of human macrophage colony stimulating factor by domestic silkworm gene engineering" & CN 1 096 541 A (UNIV NANJING), 21 December 1994 (1994-12-21)
- PHAM M ET AL: "Human interleukin-2 production in insect (Trichoplusia ni) larvae: effects and partial control of proteolysis" BIOTECHNOL. BIOENG., vol. 62, no. 2, 20 January 1999 (1999-01-20), pages 175-182, XP002164514 cited in the application
- LOUKERIS T G ET AL: "GENE TRANSFER INTO THE MEDFLY, CERATITIS CAPITATA, WITH A DROSOPHILA HYDEI TRANSPOSABLE ELEMENT" SCIENCE,AMERICAN ASSOCIATION FOR THE ADVANCEMENT OF SCIENCE,,US, vol. 270, no. 5244, 22 December 1995 (1995-12-22), pages 2002-2005, XP000941764 ISSN: 0036-8075 cited in the application
- LOUKERIS T ET AL: "Introduction of the transposable element Minos into the germ line of Drosophila melanogaster" PROCEEDINGS OF THE NATIONAL ACADEMY OF SCIENCES OF USA., vol. 92, no. 21, 10 October 1995 (1995-10-10), pages 9485-9489, XP002164515 cited in the application
- PLASTERK R H A ET AL: "Resident aliens: the Tc1/mariner superfamily of transposable elements" TRENDS IN GENETICS,NL,ELSEVIER SCIENCE PUBLISHERS B.V. AMSTERDAM, vol. 15, no. 8, 1 August 1999 (1999-08-01), pages 326-332, XP004172552 ISSN: 0168-9525
- JOWETT T ET AL: "MAMMALIAN GENES EXPRESSED IN DROSOPHILA: A TRANSGENIC MODEL FOR THESTUDY OF MECHANISMS OF CHEMICAL MUTAGENESIS AND METABOLISM" EMBO JOURNAL,GB,OXFORD UNIVERSITY PRESS, SURREY, vol. 10, no. 5, 1991, pages 1075-1081, XP000770307 ISSN: 0261-4189
- RANCOURT D E ET AL: "DIFFERENTIAL TRANSLATABILITY OF ANTIFREEZE PROTEIN MRNAS IN A TRANSGENIC HOST" BIOCHIMICA ET BIOPHYSICA ACTA. GENE STRUCTURE AND EXPRESSION,NL,ELSEVIER, AMSTERDAM, vol. 1129, no. 2, 1992, pages 188-194, XP000199687 ISSN: 0167-4781

## Description

The present invention relates to a system for the production of proteins in insect larvae. In particular, the invention encompasses the production of proteins of interest in Drosophila and Medfly larvae.

Protein production systems, in which polypeptides or proteins of interest are produced in recombinant organisms or cells, are the backbone of commercial biotechnology. The earliest systems, based on bacterial expression in hosts such as *E. coli*, have been joined by systems based on eukaryotic hosts, in particular mammalian cells in culture, insect cells both in culture and in the form of whole insects, and transgenic mammals such as sheep and goats.

Prokaryotic cell culture systems are easy to maintain and cheap to operate. However, prokaryotic cells are not capable of post-translational modification of eukaryotic proteins. Moreover, many proteins are incorrectly folded, requiring specific procedures to refold them, which adds to the cost of production.

Eukaryotic cell culture systems have been described for a number of applications. For example, mammalian cells are capable of post-translational modification, and generally produce proteins which are correctly folded and soluble. The chief disadvantages of mammalian cell systems include the requirement for specialised and expensive culture facilities, and the risk of infection, which can lead to loss of the whole culture.

Plant production systems may be used for protein expression, and may achieve high-yield production. However, transgenic plants crops are diffcult to contain, raising the risk of contamination of the environment with genetically manipulated material.

Insect cells are also used for polypeptide expression. The most widespread expression system used in insect cells is based on baculovirus vectors. A baculovirus expression vector is constructed by replacing the polyhedrin gene of baculovirus, which encodes a major structural protein of the baculovirus, with a heterologous gene, under the control of the strong native polyhedrin promoter. Cultured insect host cells are infected with the recombinant virus, and the protein produced thereby can be recovered from the cells themselves or from the culture medium if suitable secretion signals are employed. These systems also, however, suffer from problems associated with infection of the culture and the requirement for specialised culture facilities.

Organism based expression systems avoid many of the infection disadvantages and are easier to grow than cell cultures. For instance, the use of virus vectors such as baculovirus allows infection of entire insects, which have fewer requirements for special growth conditions. Large insects, such as silk moths, provide a high yield of heterologous protein. The protein can be extracted from the insects according to conventional extraction techniques.

Also known are techniques based on expression of proteins of interest in mammals such as goats and sheep, under the control of milk protein expression control sequences such that they are expressed in milk. Such techniques have great potential advantages, but are expensive due to the requirement for isolation of endogenous mammalian viruses, prions and proteins from the final product. Moreover, the cost of generating and keeping large transgenic animals is high.

The use of insect larvae, those of *Trichoplusa ni,* have been proposed for use in protein production systems (Pham et al., 1999 Biotech Bioeng 62:175-182). However, such systems have only been suggested in combination with viral vector technology based around baculoviruses.

Where proteins are intended for dietary or pharmaceutical use, the use of bacterial systems and/or viral vectors is undesirable. There is therefore a requirement in the art for a protein production system which is both robust and scaleable, as whole organism based systems are, and free from virus-based vectors, as well as inexpensive to operate in a contained environment.

### Summary of the Invention

According to a first aspect, the present invention provides a method for producing a protein of interest comprising:
(a) transforming the germline of an insect selected from the group consisting of *Bactrocera oleae* (olive fly), *Bactrocera orientalis* (oriental fruit fly), *Heliothis armigera* (cotton bollworm), *Trichoplusa ni* (cabbage looper), *Manduca sexta* (tobacco hornworm), *Lobesia botrana* (grapevine moth), *Anopheles gambiae* (mosquito), *Aedes aegypti* (yellow fever mosquito), *Glossina morsitans* (tse-tse fly), *Simulium sp.* (black fly), *Phlebotomus sp.* (sand fly), *Musca domestica* (house fly) *Drosophila melanogaster* and *Ceratitis capitata* (Medfly) with a transposon-based expression system capable of expressing the protein of interest in the larvae of the insect and secreting the protein into the haemolymph under the control of suitable control regions and signal peptides.
(b) breeding the insect to produce larvae;
(c) mass rearing the larvae; and
(d) isolating milligram quantities of the protein of interest from the haemolymph of mass-reared larvae, wherein the transposon is selected from the group consisting of *Minos, mariner, Hermes, sleeping beauty and piggyBac.*

The method according to the present invention allows the rapid production of milligram quantities of polypeptides for research purposes, the production of kilogram quantities of proteins for clinical and diagnostic applications and potentially thousands of kilograms of industrial enzymes at low cost, due to the ease with which insect larvae may be cultured using established culture techniques.

The advantages of methods according to the invention are manifold. Mass rearing of insect larvae is possible using existing technology and permits the production of polypeptide products at extremely low cost, in a controlled production environment. This facilitates regulatory approval with respect to whole organisms such as mammals, where the absence of viruses and prions must be proven before approval is given. Moreover, the method of the invention avoids the disadvantages associated with animal (including insect) cell culture, which include the higher risk of infection, and the risk of viral or prion contamination in the case of mammalian cell culture or transgenic production systems.

The term "protein" includes single-chain polypeptide molecules as well as multiple-polypeptide complexes where individual constituent polypeptides are linked by covalent or non-covalent means. The term "polypeptide" includes peptides of two or more amino acids in length, typically having more than 5, 10 or 20 amino acids.

Suitable transposons are described in more detail below.

The expression system used in the method of the invention comprises control elements which are active in insect cells, and particularly in insect larvae. Many insect control sequences, including various promoters, are active in a number of diverse species. Therefore, it is not essential that sequences derived from the insect in question be used. Inducible or constitutively active sequences may be used.

Preferably, the control sequences are inducible sequences. A preferred inducible promoter is the heat shock protein HSP70 promoter, which is induced by increasing the temperature at which the larvae are cultured, and tetracycline-inducible expression systems (Heinrich and Scott, PNAS 97:8229-8232, 2000).

In order to increase protein production, multiple expression systems may be used. These systems may be arranged with two or more coding sequences present in each system, or as multiple single systems. In either case, a balancer chromosome may be used in order to favour expression system retention and transmission to progeny during insect crossing.

An advantage of insect larval culture is that the cultures can be synchronised, such that all larvae reach the same level of maturity at the same time. This can be achieved by varying the culture temperature, since larvae develop more slowly at lower temperatures. For example, at 18 °C, larvae develop at half the rate compared to growth at 25°C.

### Brief Description of the Figures

Figure 1 shows the structure of the pMiHspGFP/HspCcW transposon, which expresses GFP and the white gene under the control of the Hsp70 promoter.
Figure 2 shows the structure of plasmid pMiAct5CGFP, which expresses GFP under the control of the Drosophila Actin 5C gene.
Figure 3 shows the expression of GFP in transgenic medfly and medfly larvae after heat shock.
Figure 4 shows constitutive expression of GFP in Drosophila flies.
Figure 5 shows the constitutive expression of GFP in adult Drosophila. The fluorescence observed is gene dose-dependent.
Figure 6 shows the expression of GFP in Drosophila larvae (top: transgenic; bottom: wildtype).
Figure 7 shows the structure of a vector for expression of a protein in larval haemolymph. LspP/L is the promoter of Drosophila or Medfly Larval Serum Protein (Lsp) gene, followed by the Lsp 5' untranslated leader (striped box) and the Lsp signal peptide (black box). The gene of interest is fused in frame to the leader peptide. HspP and HspT are heat shock 70 gene promoter and terminator, respectively. CcW is the medfly white gene, used as a dominant visible marker for detecting transformants. MiL and MiR are the ends of the Minos element.
Figure 8 is a preliminary sequence of an anti-PERV p30 antibody clone isolated from a camelid antibody library and expressed in medfly larvae.

### Detailed Description of the Invention

Although in general the techniques mentioned herein are well known in the art, reference may be made in particular to Sambrook et al., Molecular Cloning, A Laboratory Manual (1989) and Ausubel et al., Short Protocols in Molecular Biology (1999) 4th Ed. John Wiley & Sons, Inc.

### Transposons

Transposons are genetic elements which are capable of "jumping" or transposing from one position to another within the genome of a species. They are widely distributed amongst animals, including insects.

Transposons are active within their host species due to the activity of a transposase protein encoded by the elements themselves, or provided by other means - such as by injection of transposase-encoding mRNA. the use of a second coding sequence encoding transposase or the addition of transposase protein itself. Advances in the understanding of the mechanisms of transposition have resulted in the development of genetic tools based on transposons which can be used for gene transfer.

The transposable element is selected from the group consisting of *Minos, mariner, Hermes, Sleeping Beauty* and *piggyBac.*

*Minos* is a transposable element which is active in Medfly. It is described in US patent 5,840,865, which is incorporated herein by reference in its entirery. The use of *Minos* to transform insects is described in the foregoing US patent.

*Mariner* is a transposon originally isolated from *Drosophila,* but since discovered in several invertebrate and vertebrate species. The use of *mariner* to transform organisms is described in International patent application WO99/09817.

*Hermes* is derived from the common housefly. Its use in creating transgenic insects is described in US patent 5,614,398, incorporated herein by reference in its entirety.

*PiggyBac* is a transposon derived from the baculovirus host *Trichoplusia ni*. Its use for germ-line transformation of Medfly has been described by Handler et al., (1998) PNAS (USA) 95:7520-5.

Gene transfer is performed by proven transposon-mediated germ line transformation. The transposon of choice is Minos, which has been shown to function as a transgenesis vector in medfly (Loukeris et al., 1995) and Drosophila. Other transposons that are functional in medfly, such as piggyBac (McCombs et al., 1998) and can be used as alternatives. Medfly transformation methodology is well described in the literature (Loukeris et al., 1995). Briefly, circular plasmid DNA containing a transposon consisting of a gene of interest (such as erythropoietin, plus appropriate regulatory DNA sequences) flanked by the two transposon ends is co-injected into pre-blastoderm medfly embryos along with a source of transposase (a plasmid expressing transposase, in vitro-synthesised mRNA encoding transposase, or the transposase protein itself). In the early embryos, transposase interacts with the transposon ends and catalyses excision of the transposon and reintegration into chromosomes at random positions. Usually, to facilitate detection of transgenic flies, a "marker gene", such as a gene that confers a dominant, visible or otherwise selectable phenotype, is also included in the transposon. Transgenic flies are detected among the progeny of the injected flies using the marker gene phenotype and then bred to homozygosity. Several such strains, each containing an insertion at a different position in the genome, can be produced in each transformation experiment involving injection of several hundred eggs.

### Multiple Insertions

In order to increase the levels of expression, strains containing multiple insertions can be constructed by inter-breeding different transgenic strains. This procedure can be facilitated by the use of balancer chromosomes. Balancer chromosomes contain several overlapping inversions, one or more recessive lethal mutations and at least one dominant visible mutation. These chromosomes suppress recombination and can be used to keep and manipulate chromosomes carrying several mutant genes. A medtly balancer chromosome. *FiM 1*, has been described. By suppressing recombination over a large region of chromosome 5, it can be used for construction of a chromosome 5 carrying several transposon insertions.

### Choice of Insect and Promoter

Insect larvae suitable for use in the present invention are any one of *Boctrocera oleae* (olive fly), *Bactrocera orientalis* (oriental fruit fly), *Heliothis armigera* (cotton bollworm), *Trichoplusa ni* (cabbage looper). *Manduca sexta* (tobacco hornworm), *Lobesia botrana* (grapevine moth), *Anopheles gambiae* (mosquito), *Aedes aegypti* (yellow fever mosquito), *Glossina morsitans* (tse-tse fly), *Simulium sp.* (black fly), *Phlebotomus sp.* (sand fly), *Musca domestica* (house fly) Drosophila melanogaster and *Ceratitis capitata* (Medfly). Preferred, however, is the medfly.

Preferably, the promoter used is a strong promoter. Two alternative categories of promoter are available for use: inducible and constitutive promoters.

Inducible promoters include, for example, heat shock promoters. Preferably, the heat shock promoter is an insect heat shock promoter, for example the *Drosophila melanogaster hsp70* promoter, which is capable of driving the expression of genes in heterologous organisms, including medfly. The invention also encompasses the use of the medfly *hsp70* promoter (Papadimitriou et al., (1998) Insect Mol Biol 7:279-90). Alternative systems may be based on induction with the antibiotic tatracycline (Heinrich and Scott, PNAS 97:8229-8232, 2000).

Heat shock promoters are inducible by raising the temperature of the conditions under which the medfly are being cultured. For example, at 23-25°C, the *hsp70* promoter is active at low levels or not at all. This allows the insect larva to develop without stress induced by the production of a heterologous protein. At higher temperatures, however, such as 37-42°C. the *hsp70* promoter is induced and expresses the heterologous protein at a high level.

Inducible promoters may be constructed based on known inducible gene control elements. For example, inducible promoters may be constructed by combining an element responsive to a drug or hormone which may be administered in the diet. In a preferred embodiment, a human oestrogen responsive element (ERE) may be used to regulate expression of the protein of interest, as long as the insect is transformed with a second coding sequence which expresses the human oestrogen receptor.

Constitutive promoters may also be used to express the protein of interest and/or other proteins required in the insect larva. For example, the constitutive promoter may be a cytoplasmic actin promoter. The *D. Melanogaster* cytoplasmic actin promoter has been cloned (*Act5C*) and is highly active in mosquitoes (Huynh and Zieler, (1999) J. Mol. Biol. 288:13-20). Cytoplasmic actin genes and their promoters may also be isolated from other insects, including medfly.

Other examples include the cytoplasmic tubulin promoter, for instance the medfly cytoplasmic tubulin promoter.

Promoters which control secreted polypeptides may be used, together with appropriate signal sequences, to direct secretion of the protein to the haemolymph. For example, the larval serum protein promoter may be employed (Benes et al., (1995) Mol. Gen. Genet. 249(5):545-56).

### Rearing of Larvae

Mass rearing technology for medfly is highly developed. Mass rearing facilities exist that produce over 1,000 million flies per week. These flies are used for medfly pest control by the sterile insect technique: they are sterilised by exposure to radiation at the pupal stage, and then released in the field.

The life cycle of the medfly is about 25 days at 25° C and a female normally lays 100-200 eggs. As all holometabolous insects, the medfly has four distinct developmental stages: Embryonic (lasts about 2 days), larval (about 8 days), pupal (about 10 days) and adult. Sexual maturity is attained within 4-6 days of adult life. Just before pupariation, each larva is about 10 mg in mass and contains approximately 200 micrograms of protein.

For protein production, larval cultures that have been initiated at different times can be synchronised by appropriate temperature shift regimes. This is possible because growth rates depend on the temperature of the environment: at 18° C larval growth rates decrease by approximately 50%.

For laboratory scale production, the use of smaller insects such as Drosophila is preferred. Although less protein is produced per larva, the life cycle is shorter and production may be established more rapidly. For instance, the life cycle of Drosophila is 12 days, with 1mg larvae capable of each yielding 20µg of protein.

The invention is described, for the purposes of illustration only, in the following examples.

### Examples

### Reference Example 1: Production of GFP in medfly and Drosophila larvae

The gene encoding Green Fluorescent Protein from the jellyfish Aequoria victoria has been expressed in the medfly, Ceratitis capitata, and in the fruitfly Drosophila melanogaster. Transgenic Drosophila and medfly larvae, pupae and adults containing single and multiple insertions of a Minos/GFP transposon in their genome show GFP-characteristic fluorescence. Two GFP constructs are used, one with an inducible promoter (the Drosophila Hsp70) and one with a constitutive promoter (the Drosophila actin5C promoter). Drosophila and medfly Larvae with the Hsp70/GFP gene show low levels of fluorescence at the normal rearing temperature (22 degrees C) and elevated fluorescence after exposure for 1 hr at an elevated temperature (39 degrees C). Drosophila larvae with the actin5C/GFP gene show constitutive high levels of GFP fluorescence. GFP is expressed in most, if not all tissues of Drosophila and medfly. GFP protein is also detected in transgenic insects using an immunoblot assay. Comparison of several transgenic lines showed that levels of GFP expression depend on the position of integration of the transgene and on the number of transgene copies present in the genome.

### Materials and methods

Flies and DNA injection: The Drosophila yw strain and the Ceratitis capitata strain A71. a white-eves strain homozygous for the w1 gene, are used in all experiments; flies are reared at 22 degrees C under standard conditions. DNA injections of transposon plasmids along with a helper plasmid expressing transposase are performed using pre-blastoderm embryos as previously described (Loukeris et al, 1995. Science).

### Plasmid constructions and DNA analysis:

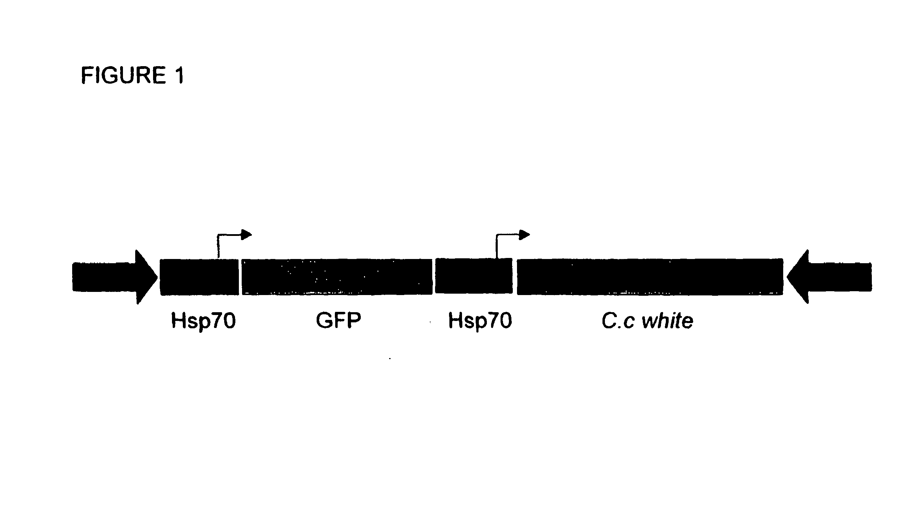

Plasmid pMiHsp70/GFP is constructed by inserting a PCR-amplified fragment containing the GFP gene into the multiple cloning site of plasmid pHSS6Hsp70PT. in the appropriate orientation. Plasmid pHSS6Hsp70PT contains, in the appropriate orientations, the promoter and the 3' mRNA trailer - polyadenylation signal (hereinbelow called 'terminator') of the Hsp70 gene of Drosophila melanogaster. Promoter and terminator are separated by a multiple cloning site. The Hsp70 promoter also contains the 5' untranslated leader sequence of the Hsp70 mRNA for increased mRNA stability. The promoter/GFP/terminator cassette is then cloned as a NotI fragment into a Minos vector, containing the wild-type cDNA of the Medfly white gene (Loukeris et al., 1995, Science). The white gene is used as a primary visible genetic marker for detection of transformants (Loukeris et al., 1995, Science). The structure of the pMiHspGFP/HspCcW transposon is shown in Figure 1.

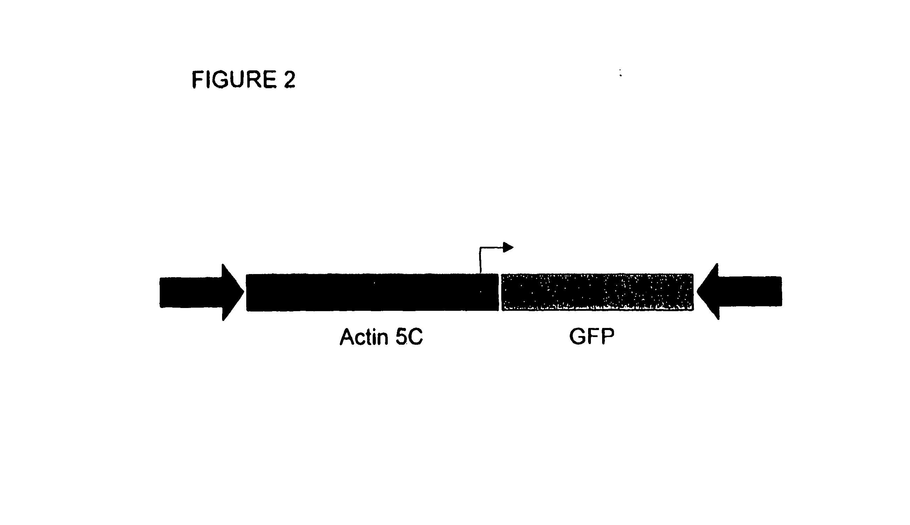

Plasmid pMiAct5CGFP (Figure 2) contains the GFP gene downstream of the Drosophila melanogaster 2kb fragment containing the promoter of the Actin5C gene, which encodes ubiquitously expressed cytoplasmic actin. Plasmid pMiAct5CGFP does not contain the white gene as a primary marker, and transformants generated with this plasmid are identified on the basis of GFP expression only (see below).

In a typical transgenesis experiment, a mixture of transposon plasmid DNA and helper plasmid DNA are co-injected into pre-blastoderm (0-1 hour post-oviposition) Drosophila or medfly embryos homozygous for the eye colour mutation white. To detect transgenic medfly, the flies derived from injected embryos (generation G0) are bred by back-crossing to the recipient strain, and their progeny are tested individually at the larval stage for expression of either the white gene (flies injected with pMiHspGFP/HspCcW) or GFP (flies injected with pMiAct5CGFP). Expression of white is detectable as a change of eye colour from white to coloured (varying form pale yellow to wild-type red). Expression of GFP is monitored by detecting GFP-specific fluorescence in the tissues of larvae after two successive 1-hour heat treatments of larvae at 39° C separated by one day, using standard epifluorescence microscopy.

Individual transgenic larvae (generation G1) are bred by back-crossing to the recipient strain and individual white or GFP-expressing progeny (G2) are inter-bred to produce homozygous progeny (G3). Intensity of eye colour or of GFP fluorescence is dependent on gene dosage. Homozygous individuals are detected, therefore, among the G3 progeny by following these phenotypes. Putative homozygous G3 individuals are interbred and their progeny is analysed by Southern blotting to determine whether they contain single or multiple insertions. Strains homozygous for single insertions are established by this procedure.

### Heat-inducible expression of GFP in Drosophila and medfly larvae

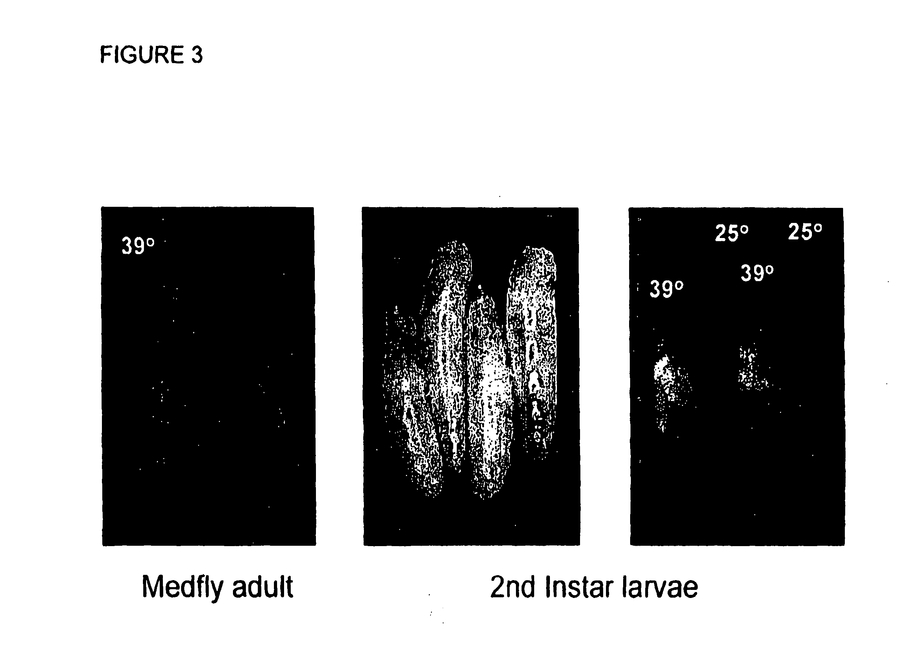

Transgenic Drosophila and medfly larvae homozygous for insertions of the MiHspGFP/HspCcW transposon grown at the standard temperature of 22-24 degrees C show low but detectable levels of GFP fluorescence compared with non-transgenic larvae. Fluorescence increases dramatically after heat shock (Figure 3).

### Constitutive expression of GFP in Drosophila

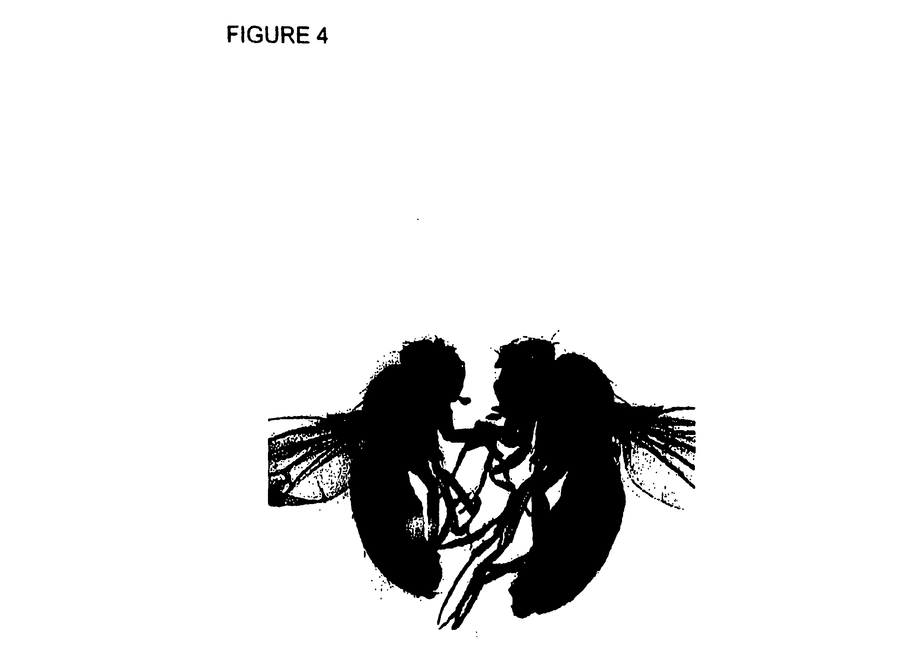

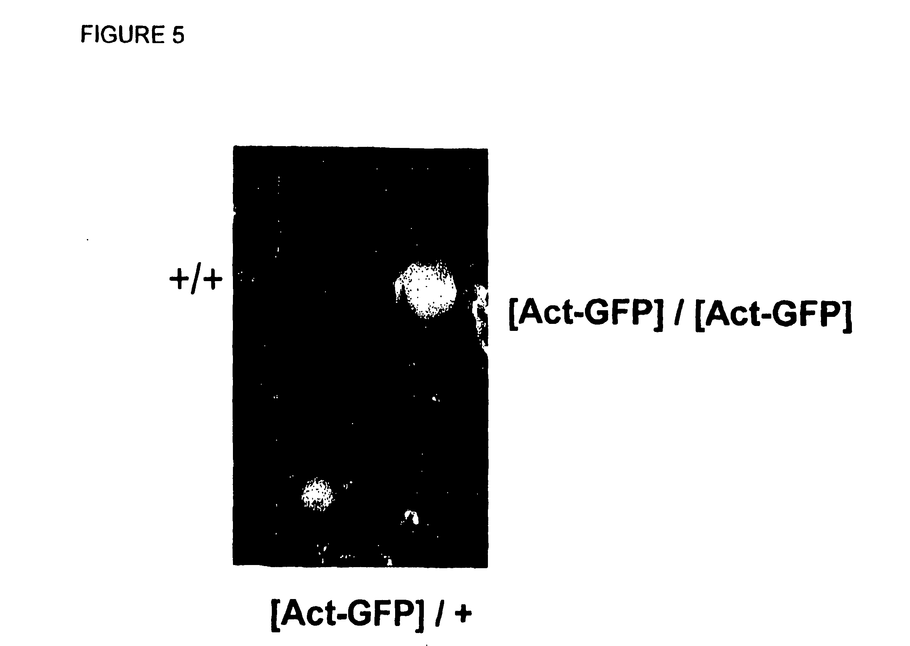

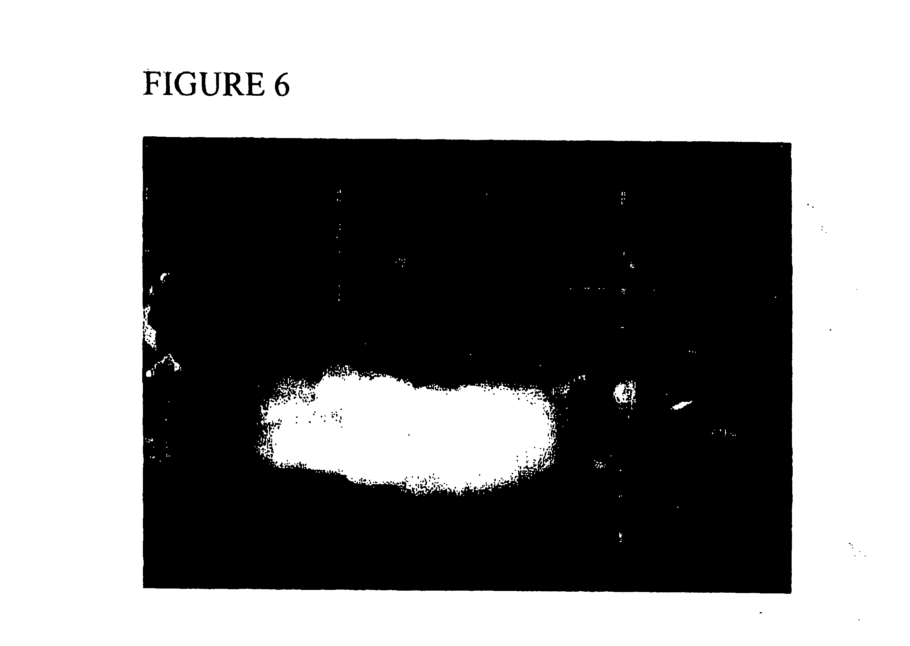

Transgenic Drosophila homozygous for insertions of the transposon MiAct5CGFP grown at the standard temperature of 22-24 degrees C show high levels of GFP fluorescence compared with non-transgenic in adult flies (Figure 4) and in larvae (Figure 6). The levels of fluorescence are gene dose dependent (Figure 5).

No GFP-expressing medfly are detected in the progeny of more than 1000 G0's injected with MiAct5CGFP. We conclude that the Drosophila actin5C promoter is a weak promoter in medfly and that a homologous actin promoter will have to be used in medfly to achieve high levels of constitutive expression of heterologous proteins.

Several Drosophila transgenic lines homozygous for MiAct5CGFP are compared to each other for expression of GFP. Although all lines demonstrated high levels of fluorescence, line-specific differences in intensity are detectable.

### Example 2: Production Of Human Growth Hormone In Medfly Larvae

A cDNA sequence encoding hGH is cloned downstream of the *D. melanogaster* promoter for the *Hsp*70 heat shock gene. The *Hsp*70 promoter also contains the 5' untranslated leader sequence of the *Hsp*70 mRNA for increased mRNA stability. The *D. melanogaster Hsp70 3*' untranslated trailer, containing a polyadenylation signal, is cloned downstream of the hGH encoding sequence. The construct is inserted in a *Minos* vector also containing a marker construct. The marker construct consists of the Green Fluorescent Protein (GFP) gene from *Aequoria victoria* driven by the *D. melanogaster Hsp70* promoter and also containing the *Hsp70 3*' region. This marker confers a visible genotype (GFP fluorescence) to transgenic organisms and is used for detecting transgenic medfly at the embryonic, larval, or adult stage. The overall structure of the complete transposon is, therefore:
*Minos* left inverted repeat - hGH expressing construct - GFP marker expressing construct *- Minos* right inverted repeat.

The transposon is constructed in *E. coli* in a BlueScript vector.

*In vitro* synthesised mRNA encoding *Minos* transposase is used as a transposase source in transgenesis experiments. The mRNA is synthesised *in vitro* using as a template an expression vector plasmid containing the transposase gene cloned downstream from a phage T7 promoter and commercially-available T7 RNA polymerase. The complete uninterrupted DNA sequence encoding *Minos* transposase is obtained by reverse transcription of the *Minos* mRNA from transgenic *D. melanogaster* expressing *Minos* transposase.

Plasmid DNA containing the transposon is co-injected with *Minos* transposase mRNA into pre-blastoderm (0-1 hour post-oviposition) embryos. Conditions for medfly embryo handling and injection have been described in the literature (Loukeris *et al*., 1995). Under these conditions, 10-20% of fertile flies derived from the injected embryos are expected to give transgenic progeny. To detect transgenic medfly, the files derived from injected embryos (generation G0) are bred by back-crossing to the recipient strain, and their progeny are tested individually at the larval stage for expression of GFP. This is done by detecting GFP-specific fluorescence in the tissues of larvae after two successive 1-hour treatments at 39°C, separated by one day, using standard epifluorescence microscopy.

Individual transgenic larvae (generation G1) are bred by back-crossing to the recipient strain and individual GFP-expressing progeny (G2) are inter-bred to produce homozygous progeny (G3). Homozygous G3 individuals are detectable by measuring GFP expression by quantitative epifluorescence microscopy. Strains homozygous for single insertions are established.

In such transgenic strains, levels of transgene expression depend not only on the promoter and on conditions of induction, but also on the point of transgene insertion. Several independently obtained strains (i.e., strains derived from different G0 flies) are tested for levels of hGH expression and those showing the highest levels are characterised further at the molecular and cytogenetic levels. Molecular characterisation consists of Southern analysis, and, for strains that will eventually be used for protein production, cloning and sequencing of the transgene. Cytogenetic characterisation is done by *in situ* hybridisation on salivary gland polytene chromosomes to determine the chromosomal point of insertion.

To construct strains with multiple insertions, appropriate crosses are performed between members of different strains. The progeny of these crosses are inter-bred, and multiply homozygous individuals recovered using GFP as a marker. Depending on the chromosomal position of insertions, strains carrying balancer chromosomes can be used to facilitate these constructions. Three to four generations are required for constructing stable strains with multiple insertions.

Mass rearing of larvae is carried out according to established procedures. Larvae are reared on a semi-dry food consisting of a bran base supplemented with yeast. Larval growth is synchronised by appropriate temperature shifts and third-instar larvae treated at 39-41°C for the time required for maximal induction of the transgene. Late third instar larvae move away from food, in search of a place to pupariate. This behaviour is used to harvest larvae from the food for mass fly production in mass rearing facilities; it is also applied to the harvesting of food-free larvae for protein production and purification.

Medfly larvae are washed with chilled saline and then homogenised in the presence of protease inhibitors, hGH is purified from the homogenate according to standard procedures.

### Example 3. Production of a camelid antibody in medfly larvae

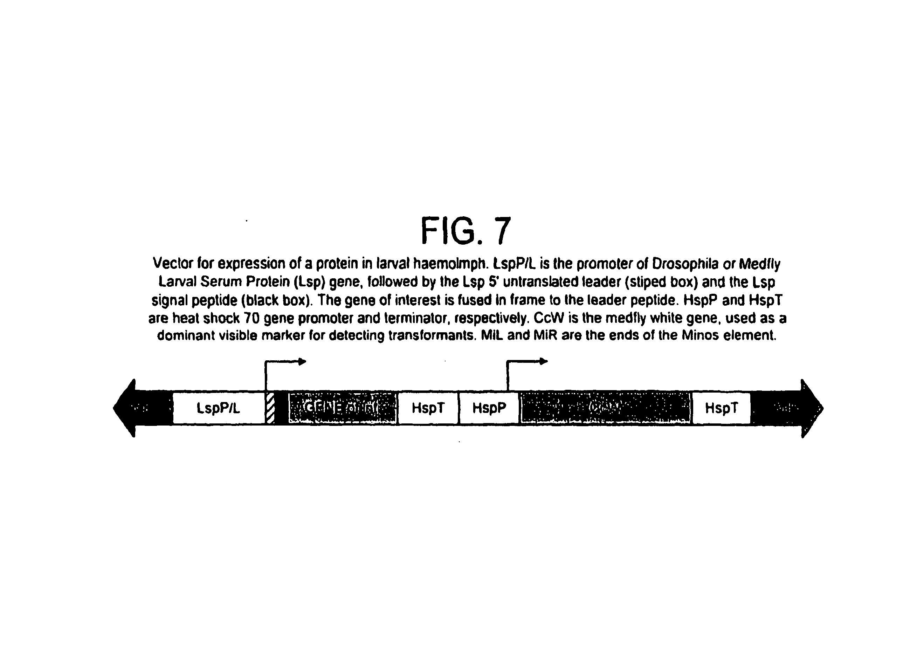

A vector designed for expression of a protein in larval haemolymph is shown in Figure 7. LspP/L is the promoter of Drosophila or Medfly Larval Serum Protein (Lsp) gene (Benes et al., (1995) Mol. Gen. Genet. 249(5):545-56), followed by the Lsp 5' untranslated leader (striped box) and the Lsp signal peptide (black box). The gene of interest, in this case the camelid antibody gene, is fused in frame to the leader peptide. HspP and HspT are heat shock 70 gene promoter and terminator, respectively. CcW is the medfly white gene, used as a dominant visible marker for detecting transformants. MiL and MiR are the ends of the Minos element.

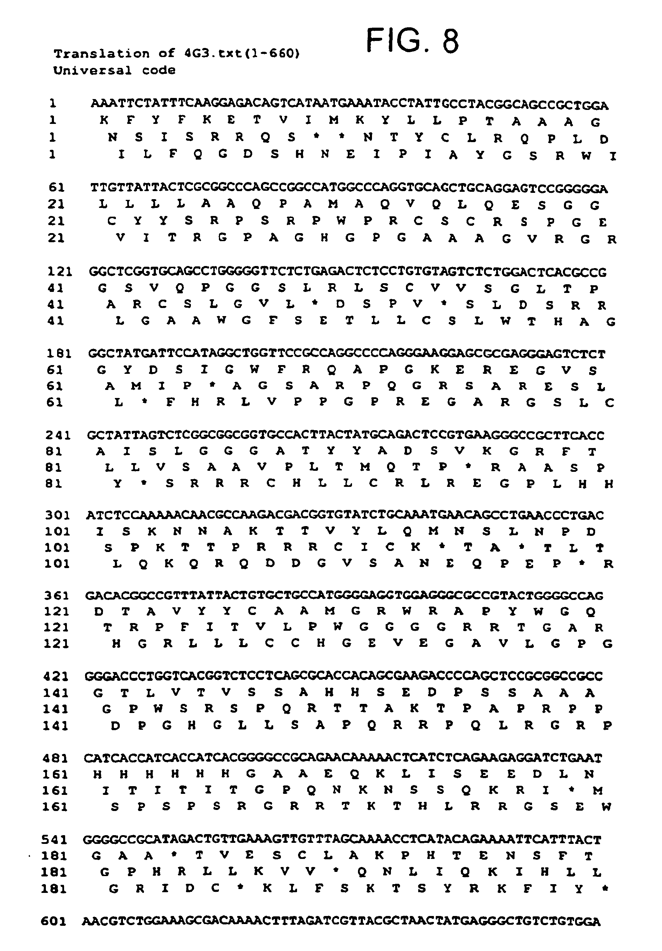

A camelid antibody is isolated from a phage expression library (Unilever). The antibody is specific for the porcine retrovirus PERV (porcine endogenous retrovirus) and recognises the p30 component of the PERV gag proteins (the viral core proteins). A p30 clone is expressed in the expression vector pHEN1 in order to obtain antigen for screening the antibody library, and antibody clones selected accordingly. A preliminary sequence and translation of the antibody used in this experiment is set forth in Figure 8.

Transgenic flies are identified by white expression, as in Example 1. Antibody expression is detected in the haemolymph of transgenic fly larvae.

Antibody is purified by homogenising larval extract and purification by Protein A column chromatography. Immunoglobulins bind to Protein A at pH 8.6 and elute from the column at pH 4.3. Thus elution is done by lowering the pH of the Protein A column. Protein A agarose (0.25 g; Sigma) is swollen in Tris-buffered saline (0.05 M Tris 0.15 M NaCl, pH 8.6) and then packed into a column (the bed volume is 1 ml). The culture supernatant is adjusted to pH 8.6 by adding dilute NaOH and is then centrifuged at 600 g for 30 min at 4 °C. After the sample is loaded, the Protein A column is washed with Tris-buffered saline, pH 8.6, until no proteins are eluted from the column. Then, step elutions are carried out with PBS (0.05 M phosphate/0.15 M NaCl, pH 7.0), citrate-buffered saline (0.05 M citrate/0.15 M NaCl, pH 5.5) and acetate-buffered saline (0.05 M acetate/0.15 M NaCl, pH 4.3) until the antibody is eluted. Fractions contributing to the peak of A 280 are pooled, dialysed in 0.01xPBS, lyophilised, redissolved in 500 µl of PBS and stored at -70 °C. The purity of the protein peak is analysed by SDS/PAGE. The column is regenerated by washing with glycine-buffered saline (0.05 M glycine/HCl/0.15 M NaCl, pH 2.3) followed by Tris-buffered saline (pH 8.6, containing 0.02% NaN₃).

An ELISA assay is performed using immobilised p30 antigen, produced as above, and a labelled murine anti-camelid monoclonal antibody. The specificity of the antibody eluted from medfly larvae for PERV p30 is thus confirmed.

### Example 4. Production of human α-glucosidase in medfly larvae

A heat-shock inducible transposon expression system in accordance with Example 1 is constructed, using the human α-glucosidase gene (GenBank GI:182907) in place of the GFP gene.

A mixture of transposon plasmid DNA and helper plasmid DNA. as per Example 1, is co-injected into pre-blastoderm (0-1 hour post-oviposition) Drosophila or medfly embryos homozygous for the eye colour mutation white. To detect transgenic medfly, the flies derived from injected embryos (generation G0) are bred by back-crossing to the recipient strain, and their progeny are tested individually at the larval stage for expression of the white gene. Expression of white is detectable as a change of eye colour from white to coloured (varying form pale yellow to wild-type red).

Individual transgenic larvae (generation G1) are bred by back-crossing to the recipient strain and individual white progeny (G2) are inter-bred to produce homozygous progeny (G3). Intensity of eye colour is dependent on gene dosage. Homozygous individuals are detected, therefore, among the G3 progeny by following these phenotypes. Putative homozygous G3 individuals are interbred and their progeny is analysed by Southern blotting to determine whether they contain single or multiple insertions. Strains homozygous for single insertions are established by this procedure.

### Heat-inducible expression of α-glucosidase in Drosophila and medfly larvae

Transgenic Drosophila and medfly larvae homozygous for insertions of the MiHsp α-glucosidase /HspCcW transposon grown at the standard temperature of 22-24 degrees C show low but detectable levels of α-glucosidase compared with non-transgenic larvae in the fat pads, as assessed by immunoquantification according to Umapathysivam et al., Clin Chem 2000 46(9):1318-25. The amount of α-glucosidase increases markedly after heat shock.

## Claims

1. A method for producing a protein of interest comprising:
(a) transforming the germline of an insect selected from the group consisting of *Bactrocera oleae* (olive fly), *Bactrocera orientalis* (oriental fruit fly), *Heliothis armigera* (cotton bollworm), *Trichoplusa ni* (cabbage looper), *Manduca sexta* (tobacco hornworm), *Lobesia botrana* (grapevine moth), *Anopheles gambiae* (mosquito), *Aedes aegypti* (yellow fever mosquito), *Glossina morsitans* (tse-tse fly), *Simulium sp.* (black fly), *Phlebotomus sp.* (sand fly), *Musca domestica* (house fly) *Drosophila melanogaster* and *Ceratitis capitata* (Medfly) with a transposon-based expression system capable of expressing the protein of interest in the larvae of the insect and secreting the protein into the haemolymph under the control of suitable control regions and signal peptides.
(b) breeding the insect to produce larvae;
(c) mass rearing the larvae; and
(d) isolating milligram quantities of the protein of interest from the haemolymph of mass-reared larvae, wherein the transposon is selected from the group consisting of *Minos, mariner, Hermes, sleeping beauty and piggyBac.*

2. A method according to claim 1, wherein transforming the insect comprises the steps of
a) providing a transposable element which encodes a transposase protein which is active in the insect;
b) modifying the transposable element by inserting a coding sequence encoding the protein of interest; and
c) transforming the target insects with the modified transposable element.

3. A method according to any proceeding claim, wherein the expression system comprises a coding sequence encoding the protein of interest, under the control of an inducible transcriptional control element which is operative in the insect larvae.

4. A method according to claim 3, wherein the inducible control system is regulatable by tetracycline or oestrogens.

5. A method according to any one of claims 1 to 2, wherein the expression system comprises a coding sequence encoding the protein of interest, under the control of a constitutive transcriptional control element which is operative in the insect larvae.

6. A method according to claim 5, wherein the constitutive control element is selected from the group consisting of the cytoplasmic actin promoter, such as the *Drosophila Act5C* promoter, and the cytoplasmic tubulin promoter.

7. A method according to claim 3, wherein the inducible expression control sequence is a heat shook promoter, such as the *Drosophila* or the medfly *hsp70* promoter.

8. A method according to any preceding claim, wherein the insect is transformed with two or more expression systems, or an expression system comprising two or more coding sequences.

9. A method according to any preceding claim, wherein the protein of interest is selected from the group consisting of an enzyme, a cytokine, a hormone or proteinaceous pharmaceutical.

10. A method according to claim 9 wherein the insect is *Ceratitis capitata*.

11. A method according to any preceding claim, wherein the larval cultures are synchronised by manipulation of culture temperatures.

## Patentansprüche

1. Verfahren zur Herstellung eines interessierenden Proteins, bei dem man
(a) die Keimbahn eines Insektes, ausgewählt aus der Gruppe bestehend aus *Bactrocera oleae* (Olivenfliege), *Bactrocera orientalis* (orientalische Fruchtfliege), *Heliothis armigera* (Baumwollkapselwurm), *Trichoplusa ni* (Kohlspannerraupe), *Manduca sexta* (Tabakschwärmerraupe), *Lobesia botrana* (Weinmotte), *Anopheles gambiae* (Moskito), Aedes aegypti (Gelbfiebermoskito), *Glossina morsitans* (Tse-Tse-Fliege), *Simulium sp.* (schwarze Fliege), *Phlebotomus sp.* (Sandfliege), *Musca domestica* (Stubenfliege), *Drosophila melanogaster* und *Ceratitis capitata* (Mittelmeerfruchtfliege), mit einem auf einem Transposon basierenden Expressionssystem, welches in der Lage ist, das interessierende Protein in den Larven des Insektes zu exprimieren und das Protein unter der Kontrolle von geeigneten Kontrollbereichen und Signalpeptiden in die Hämolymphe abzusondern, transformiert,
(b) das Insekt für eine Produktion von Larven kultiviert,
(c) die Larven massenweise züchtet und
(d) das interessierende Protein in Milligramm-Mengen aus den massengezüchteten Larven isoliert,
wobei das Transposon aus der Gruppe ausgewählt ist, bestehend aus *Minos, mariner, Hermes, sleeping beauty* und *piggyBac.*

2. Verfahren nach Anspruch 1, wobei das Transformieren des Insektes die Stufen umfaßt, bei denen man
a) ein transponierbares Element bereitstellt, welches ein Transposase-Protein codiert, das in dem Insekt aktiv ist,
b) das transponierbare Element durch Einsetzen einer codierenden Sequenz, welche das interessierende Protein codiert, modifiziert und
c) die Zielinsekten mit dem modifizierten transponierbaren Element transformiert.

3. Verfahren nach einem der vorangegangenen Ansprüche, wobei das Expressionssystem eine codierende Sequenz, welche das interessierende Protein codiert, unter der Kontrolle eines induzierbaren Transkriptionskontrollelementes, welches in den Insektenlarven funktionsfähig ist, umfaßt.

4. Verfahren nach Anspruch 3, wobei das induzierbare Kontrollsystem durch Tetracyclin oder Östrogene regulierbar ist.

5. Verfahren nach einem der Ansprüche 1 bis 2, wobei das Expressionssystem eine codierende Sequenz, welche das interessierende Protein codiert, unter der Kontrolle eines konstitutiven Transkriptionskontrollelementes, welches in den Insektenlarven funktionsfähig ist, umfaßt.

6. Verfahren nach Anspruch 5, wobei das konstitutive Kontrollelement aus der Gruppe ausgewählt ist, bestehend aus dem zytoplasmatischen Actinpromotor, wie dem *Drosophila Act5*C-Promotor, und dem zytoplasmatischen Tubulinpromotor.

7. Verfahren nach Anspruch 3, wobei die induzierbare Expressionskontrollsequenz ein Hitzeschockpromotor ist, wie der *Drosophila-* oder der Mittelmeerfruchtfliege-*hsp70-*Promotor.

8. Verfahren nach einem der vorangegangenen Ansprüche, wobei das Insekt mit zwei oder mehr Expressionssystemen oder einem Expressionssystem, welches zwei oder mehr codierende Sequenzen umfaßt, transformiert wird.

9. Verfahren nach einem der vorangegangenen Ansprüche, wobei das interessierende Protein aus der Gruppe ausgewählt ist, bestehend aus einem Enzym, einem Zytokin, einem Hormon oder einem proteinartigen Arzneimittel.

10. Verfahren nach Anspruch 9, wobei das Insekt *Ceratitis capitata* ist.

11. Verfahren nach einem der vorangegangenen Ansprüche, wobei die Larvenkulturen durch Manipulation der Kulturtemperaturen synchronisiert werden.

## Revendications

1. Procédé pour la production d'une protéine intéressante, comprenant :
(a) la transformation de la lignée germinale d'un insecte choisi dans le groupe consistant en *Bactrocera oleae* (mouche de l'olive), *Bactrocera orientalis* (mouche orientale des fruits), *Heliothis armigera* (chenille des épis de maïs), *Trichoplusa ni* (chenille arpenteuse du chou), *Manduca sexta* (ver du tabac), *Lobesia botrana* (eudemis), *Anopheles gambiae* (moustique), *Aedes aegypti* (moustique de la fièvre jaune), *Glossina morsitans* (mouche tsé-tsé), *Simulium sp.* (simulie), *Phlebotomus sp.* (phlébotome), *Musca domestica* (mouche domestique), *Drosophila melanogaster* et *Ceratitis capitata* (mouche méditerranéenne des fruits) avec un système d'expression à base de transposon capable d'exprimer la protéine intéressante chez les larves de l'insecte et la sécrétion de la protéine dans l'hémolymphe sous le contrôle de régions de régulation et peptides signaux convenables ;
(b) la reproduction de l'insecte pour produire des larves ;
(c) l'élevage en masse des larves ; et
(d) l'isolement de quantités en milligrammes de la protéine intéressante à partir des larves élevées en masse, dans lequel le transposon est choisi dans le groupe consistant en *Minos, mariner, Hermes, sleeping beauty* et *piggyBac.*

2. Procédé suivant la revendication 1, dans lequel la transformation de l'insecte comprend les étapes consistant:
a) à fournir un élément transposable qui code pour une protéine transposase qui est active chez l'insecte ;
b) à modifier l'élément transposable en insérant une séquence codante qui code pour la protéine intéressante; et
c) à transformer les insectes cibles avec l'élément transposable modifié.

3. Procédé suivant l'une quelconque des revendications précédentes, dans lequel le système d'expression comprend une séquence codante qui code pour la protéine intéressante, sous le contrôle d'un élément de régulation de transcription inductible qui est opérationnel chez les larves d'insecte.

4. Procédé suivant la revendication 3, dans lequel le système de régulation inductible est régulable par la tétracycline ou des oestrogènes.

5. Procédé suivant l'une quelconque des revendications 1 et 2, dans lequel le système d'expression comprend une séquence codante qui code pour la protéine intéressante, sous le contrôle d'un élément de régulation de transcription constitutif qui est opérationnel chez les larves d'insecte.

6. Procédé suivant la revendication 5, dans lequel l'élément de régulation constitutif est choisi dans le groupe consistant en le promoteur d'actine cytoplasmique, tel que le promoteur Act5C de *Drosophila,* et le promoteur de tubuline cytoplasmique.

7. Procédé suivant la revendication 3, dans lequel la séquence de régulation d'expression inductible est un promoteur de choc thermique, tel que le promoteur *hsp70* de *Drosophila* ou de mouche méditerranéenne des fruits.

8. Procédé suivant l'une quelconque des revendications précédentes, dans lequel l'insecte est transformé avec deux ou plus de deux systèmes d'expression, ou avec un système d'expression comprenant deux ou plus de deux séquences codantes.

9. Procédé suivant l'une quelconque des revendications précédentes, dans lequel la protéine intéressante est choisie dans le groupe consistant en une enzyme, une cytokine, une hormone et un agent pharmaceutique protéique.

10. Procédé suivant la revendication 9, dans lequel l'insecte est *Ceratitis capitata.*

11. Procédé suivant l'une quelconque des revendications précédentes, dans lequel les élevages de larves sont synchronisés en agissant sur les températures d'élevage.
